# EUROPEAN PATENT SPECIFICATION

(11) **EP 1 951 374 B1**
(45) Date of publication and mention of the grant of the patent: **01.03.2017**
(21) Application number: 06808626.3
(22) Date of filing: 22.11.2006
(51) Int. Cl.: A61P 31/02, A61K 31/5375, A61K 9/00

(54) **TREATMENT OF SUB-GINGIVAL POCKET INFECTIONS**
BEHANDLUNG VON INFEKTIONEN DER ZAHNFLEISCHTASCHEN
TRAITEMENT D'INFECTIONS DE POCHES SUBGINGIVALES

(30) Priority: 22.11.2005 GB 0523745; 09.01.2006 GB 0600324
(43) Date of publication of application: 06.08.2008
(60) Divisional application: 16199408.2
(73) Proprietor: Maelor Laboratories Limited, Chippenham Wiltshire SN15 2BB (GB); Merial, Inc., Dulluth GA 30095 (US)
(72) Inventor: PERSSON, Gosta, Rutger, CH-3011 Bern (CH)
(74) Representative: D Young & Co LLP
(86) International application number: PCT/GB2006/004348
(87) International publication number: WO 2007/060413

(56) References cited:
- WO-A-92/07548
- WO-A-92/08442
- SCANDINAVIAN JOURNAL OF DENTAL RESEARCH, COPENHAGEN, DK, vol. 102, no. 1, 1994, pages 17-25, XP008067770 ISSN: 0029-845X
- ORAL MICROBIOLOGY AND IMMUNOLOGY, MUNKSGAARD, COPENHAGEN, DK, vol. 8, no. 1, 1993, pages 36-41, XP008067768 ISSN: 0902-0055
- D Etienne: "Locally delivered antimicrobials for the treatment of chronic periodontitis", ORAL DISEASES, vol. 9, no. s1, 1 June 2003 (2003-06-01), pages 45-50, XP055219986, GB ISSN: 1354-523X, DOI: 10.1034/j.1601-0825.9.s1.8.x

## Description

### Field of the Invention

The present invention relates to the use in a treatment of sub-gingival infection.

### Background to the Invention

Gum disease is a major cause of ill-health, worldwide, and commonly leads to tooth decay. The two major forms of gum disease, gingivitis and periodontitis, result from bacterial plaque formation in the oral cavity, in particular on the teeth along the margin of the gums.

A sub-gingival infection begins when bacteria at the tooth-gingiva boundary cause the gingival tissue (i.e. the gums) to separate from the tooth, forming a sub-gingival pocket. Bacteria collect in this pocket, causing gingival swelling that traps bacteria in the sub-gingival pocket. Further inflammation exacerbates the infection and eventually both gingival tissue and bone are destroyed. Treating sub-gingival infections is particularly problematic as the infecting bacteria are hard to access and it is difficult to apply anti-bacterial agents to the site of infection. Furthermore, there is thought to be a flow of gingival fluids out of the sub-gingival pocket, which hinders entry of any therapeutic agent into the site of infection.

Sub-gingival infections are characteristic of a number of oral infections, including adult and juvenile chronic periodontitis, early-onset periodontitis, necrotizing ulcerative periodontal diseases and acute pericoronitis.

The anti-bacterial agent chlorhexidine is used to treat oral infections. When used to treat sub-gingival pocket infections, chlorhexidine suffers unsightly staining and a loss of taste and further suffers from low patient compliance due to an unpleasant stinging sensation that occurs on its application. This can be extremely painful.

Infections in sub-gingival pockets are often characterized by the presence of a combination of bacterial species known as "The Red Complex". This comprises *Tannerella forsythensis, Porphyromonas gingivalis* and *Treponema denticola* and it is thought that the components of the red complex are synergistic (see for example, Holt and Ebersole, Periodontol 2000. 2005;38:72-122). *Actinomyces* species such as *Actinobacillus actinomycetemcomitans* are also implicated in oral (sub-gingival) infections.

Changes in the bacterial flora, and the interactions between different bacterial species, are thought to influence the progression of oral disease (see for example Socransky and Haffajee, Periodontol 2000. 2005;38:135-87).

*Actinobacillus actinomycetemcomitans* is a non-motile, gram-negative, capnophilic, fermentative coccobacillus that has been implicated in the aetiology and pathogenesis of juvenile and adult periodontitis as well as systemic infections. *Actinobacillus actinomycetemcomitans* has serotypes a-e. Strain Y4 is serotype b. It has been shown that antibodies reactive to *Actinohacillus actinomycetemcomitans* serotype b (i.e. strain Y4) lipopolysaccharide correlate with reduction of attachment loss in generalised early onset peridontitis patients (Califano J.V. et al, Infection and Immunity, Sept. 1996, p.3908-3910). Most of the antibody reactive with *Actinobacillus actinomycetemcomitans* serotype b LPS is IgG2 (Lu et al, Infect Immun 1993 Jun; 61 (6): 2400-7), and serum IgG2 is decreased in smokers (Quinn, S. et al, Infect, Immun. 64:2500-2505). It should be noted that *Actinobacillus actinomycetemcomitans* is sometimes referred to in the art as *Aggregatibacter actinomycetemcomitans.*

Besides periodontal infections, *Actinobacillus actinomycetemcomitans* is occasionally isolated from severe systemic infections (Van Winkelhoff A.J. and Slots J. Periodontol. 2000 1999;20:122-135).

*Treponema denticola* infection has been proven to be closely associated with periodontal diseases such as early onset periodontitis, necrotizing ulcerative gingivitis and acute pericoronitis (Chan EC, McLaughlin R. Oral Microbiol Immunol 2000; 15: 1-9; Takeuchi Y, Umeda M, Sakamoto M, et al. J Periodontol 2001;72: 1354-1363).

Particular attention has been paid recently to the clinical significance of coinfection of periodontal bacterial pathogens *P. gingivalis, A. actinomycetemcomitans* and *T. denticola* as well as other sub-gingival bacteria form a mixed infection, which causes more serious periodontal destruction than a single infection (Langendijk-Genevaux PS et al. J Clin Periodontol 2001;28:1151-1157; Shiloah J, et al. J Periodontol 2000;71:562-567).

The morpholino compound commonly referred to as Delmopinol is known to be useful in treating and preventing dental plaque and mild gingivitis, as part of a normal oral health routine (see, for example, Collaert et al, Scand J Dent Res 1994; 102: 17- 25).

The microbiology of early supragingival plaque development after delmopinol treatment is disclosed by Collaert et al, Oral Microbiol Immunol 1993:8:36-41. Improved anti-plaque compositions comprising a combination of a morpholino amino alcohol and a chelating agent are disclosed by WO-A-92/07548. Improved anti-plaque compositions comprising a combination of a morpholino amino alcohol and an anti-microbial agent are disclosed by WO-A-92/08442.

Current treatments for sub-gingival infection, for example in chronic periodontitis, commonly involve surgical intervention, to remove the infected tissue and bacterial plaque from the sub-gingival pocket and to reduce the depth of the pocket. Even if open surgery is not involved, sub-gingival scaling and root planing is a standard treatment; locally delivered antiseptics such as anti-bacterial-soaked pads may also be inserted into the sub-gingival pocket (see for example Etienne, Oral Dis. 2003;9 Suppl 1:45-50). These treatments are expensive, time-consuming, inconvenient and painful. To date, simple anti-bacterial treatments have not been successful in treating sub-gingival infections. There is a clear need for an improved method of clearing an infection from a sub-gingival pocket.

### Summary of the invention

The present invention is based on the surprising discovery that a compound of formula (I) is efficacious in treating infection of a sub-gingival pocket that is 4mm deep or more. Sub-gingival infections commonly present in a number of periodontal diseases, and a compound of formula (I) can be used to treat these diseases. This finding is surprising, as compounds of formula (I) are known to have only very weak anti-microbial properties and would therefore not be expected to be effective in treating a well-established infection.

According to a first aspect of the invention, a morpholino compound having the general formula (I) 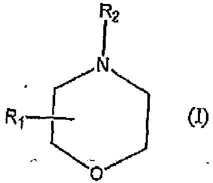 wherein R1 is a straight or branched alkyl group containing 8 to 16 carbon atoms at the 2- or 3- position of the morpholino ring, and R2 is a straight or branched alkyl group containing 2 to 10 carbon atoms, substituted with a hydroxy group except in the alpha-position, or a pharmaceutically acceptable salt thereof is used in the manufacture of a medicament for the treatment of an infected sub-gingival pocket wherein the sub-gingival pocket is 4mm deep or more.

According to a second aspect of the invention, a kit for use in treating an infection in a sub-gingival pocket that is 4mm deep or more comprises a compound having the general formula (I) and instructions that the compound is to be used for treatment of the infection.

### Description of the Drawings

The invention is described with reference to the following drawings, wherein:
Figure 1 illustrates the sub-gingival reduction in bacterial load, in four selected sub-gingival pockets, after a 14 day course of delmopinol rinsing (once daily); and
Figure 2 illustrates the reduction of *Treponema denticola* and *Aggregatibacter actinomycetemcomitans* after the 14 day course of delmopinol treatment.

### Detailed Description of the Invention

A morpholino compound of formula (I) can be used to treat sub-gingival pockets, that are 4mm deep or more, infected with bacteria. A morpholino compound according to the invention has the general formula (I) 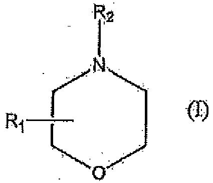 wherein R1 is a straight or branched alkyl group containing 8 to 16 carbon atoms at the 2- or 3- position of the morpholino ring, and R2 is a straight or branched alkyl group containing 2 to 10 carbon atoms, substituted with a hydroxy group except in the alpha-position, or pharmaceutically acceptable salts thereof. In a preferred embodiment, the sum of the carbon atoms in the groups R1 and R 2 of the morpholino compound is at least 10, preferably between 10 and 20. In a further preferred embodiment, the R 2 group terminates with the hydroxy group.

The claimed morpholino compounds are known *per se* and can be manufactured by any known method, for example that disclosed in US5,082,653 and WO90/14342.

The preferred morpholino compound for use in the invention is 3-(4-propyl-heptyl)-4-(2-hydroxyethyl)morpholine, which is commonly known as Delmopinol (CAS No. 79874-76-3).

The morpholino compounds can be used in their free base form or as a pharmaceutically acceptable salt thereof. Examples of pharmaceutically acceptable salts are the salts of acids such as acetic acid, phosphoric acid, boric acid, hydrochloric acid, maleic acid, benzoic acid, citric acid, malic acid, oxalic acid, tartaric acid, succinic acid, glutaric acid, gentisic acid, valeric acid, gallic acid, beta-resorcyclic acid, acetyl salicylic acid, salicylic acid, perchloric acid, barbituric acid, sulfanilic acid, phytic acid, p-nitro benzoic acid, stearic acid, palmitic acid, oleic acid, myristic acid, lauric acid and the like. The most preferred salts are those of hydrochloric acid. A preferred compound is delmopinol hydrochloride (CAS No. 98092-92-3).

As used herein, the term "infection" is to be given its usual meaning in the art, i.e. the detrimental colonization of a cell, tissue, organ or body by a foreign species. According to the current invention, the foreign species are bacteria.

The site of the infection is a "sub-gingival pocket". The term "sub-gingival pocket" is known in the art to refer to a pit or hollow that is formed between a tooth and the gingival tissue associated with that tooth. Sub-gingival pockets are usually a symptom of advanced, chronic or severe forms of gingivitis. The pocket is usually formed due to the gingival tissue separating from the tooth in response to an initially mild bacterial infection at the tooth-gingival boundary. A small, for example less than 1mm deep pocket (measured from the apex of the pocket to the edge of the gingival tissue), may not require anti-microbial treatment as a normal oral health routine may suffice, e.g. brushing may remove the bacteria. However, sub-gingival pockets can grow up to 12mm deep or more. The more severe pockets, for example deeper than 3 mm deep or more, for example 4, 5, 6, 7 or 8 mm deep or more are likely to require anti-microbial treatment. According to the present invention, a bacterial infection in a sub-gingival pocket at least 4 mm deep is treated. These deep pockets are likely to be caused by chronic periodontal infections. Using conventional methods, an infection in these deeper pockets is likely to be difficult to treat without surgical intervention.

A further advantage of using a compound of formula (I) to treat a sub-gingival infection is that, surprisingly, administration is substantially pain free.

According to the current invention, a compound of formula (I) is used to treat an existing sub-gingival infection, wherein the sub-gingival pocket is 4 mm deep or more. This is separate from the previously known use of delmopinol as a prophylactic agent to prevent an infection arising due to the ability to prevent supra-gingival plaque formation and mild gingivitis. The compound is administered to a patient that presents with infected sub-gingival pockets. Contacting the oral cavity, and therefore the sub-gingival pockets, with a compound of formula (I) reduces the number of bacteria in the infected pocket and allows the gingival tissue to recover. In a preferred embodiment, no further treatment is required, i.e. the gingival tissue grows back to an acceptable level thereby "closing" the pocket. Application of the compound of formula (I) to the site of infection does not require the intervention of a qualified medical or dental practitioner, such as a dental hygienist or periodontist.

The current invention is applicable equally in the fields of human and animal medicine, i.e. veterinary applications are within the scope of the invention. In the veterinary embodiment, treatment of pets including cats and dogs, and treatment of farm animals including cattle and swine, are preferred embodiments.

A compound of formula (I) is useful in treating sub-gingival infections and, surprisingly, it can access the sub-gingival site of infection. It has been found that a compound of formula (I) results in a significant reduction of *T. denticola* levels (see example 1). Compounds of formula (I) are therefore surprisingly effective in reducing levels of bacteria that are components of the "Red Complex", which is a characteristic of sub-gingival microflora colonisation.

It has been also been found that a compound of formula (I) almost eliminates *A. actinomycetemcomitans* (see example 1). This bacterium is implicated in the development of the red complex and is often found in infected sub-gingival pockets.

A compound of formula (I) can be used to alter the level of specific bacteria, preferably *T. denticola* and/or *A. actinomycetemcomitans* (*A. actinomycetemcomitans* is also sometimes referred to in the art as *Aggregatibacter actinomycetemcomitans*) in the oral cavity, preferably at an infected sub-gingival site.

Without wishing to be bound by theory, it is thought that the Red complex is particularly problematic because the bacterial species within the complex interact and create a stubborn infection (see, for example, Langendijk-Genevaux *et al,* supra). Therefore reducing the level of at least one member of the Red complex will reduce the pathogenicity of this complex of bacteria and therefore effectively treat a sub-gingival infection.

Sub-gingival pocket infections commonly occur in periodontal diseases such as adult and juvenile chronic periodontitis, early-onset periodontitis, necrotizing ulcerative periodontal diseases and acute pericoronitis. It is possible to treat other symptoms of these diseases, such as infection of the (supra-gingival) tooth surface and tongue, using conventional techniques. Even if such conventional treatments are successful, the sub-gingival pocket infection is likely to remain but on a lower level. Therefore, a compound of formula (I) can be used to treat these diseases when the problematic symptom is a sub-gingival infection. Preferably, chronic periodontitis is treated by contacting the infected sub-gingival area with a compound of formula (I).

Use of a compound of formula (I) to treat a sub-gingival infection will also have a cosmetic benefit, i.e. infection-free gingival tissue is considered to be aesthetically pleasing.

The compound of formula (I) may be brought into contact with the oral cavity in a conventional way, in any suitable form or amount that achieves the desired effect, i.e. reduction of infection of a sub-gingival pocket. Preferably, the compound of formula (I) is in the form of a mouthwash, toothpaste, gel, dentifrice, gum or other similar preparation that will be apparent to the skilled person. Most preferably, the compound is in the form of an aqueous mouthwash. This can be applied to the oral cavity by the patient, without the need for medical supervision. The compound of formula (I) has the surprising ability to enter the sub-gingival pocket and treat the infection. Preferably, the mouthwash is held in the mouth for at least 5 seconds, preferably greater than 10 seconds, for example one minute or more, to allow the compound of formula (I) to access the site of infection.

The compound of formula (I) can be added to an existing toothpaste, gum, gel or mouthwash formulation. The compound of formula (I) may be added in combination with at least one anti-microbial, preferably anti-bacterial, agent. Suitable agents include the antibiotics tetracycline, doxycylcine and ampicillin. Other agents suitable for treating oral infections will be apparent to one skilled in the art. Alternatively, the compound of formula (I) is the only agent that treats the sub-gingival pocket infection in the preparation with which the oral cavity is contacted.

The compound of formula (I) may be added in combination with at least one anti-inflammatory agent. Anti-inflammatory agents are well known in the art and any may be used. Preferably, the anti-inflammatory agent is a non-steroidal anti-inflammatory drug (NSAID), such as aspirin (acetylsalicylic acid) or ibuprofen. In an alternative embodiment, a steroidal anti-inflammatory agent, for example cortisone, may be used. The compound of formula (I) may be added in combination with at least one anti-inflammatory agent and at least one other anti-microbial agent.

For the avoidance of doubt, agents in addition to a compound of formula (I) can be added to a composition for treating sub-gingival infection, for example the agents disclosed above can be included. However, additional agents are not required and, in a preferred embodiment, no additional agents that have an antimicrobial, therapeutic or other pharmacological effect, or which enhance the anti-infection effect of the compound of formula (I), such as chelating agents, are included in the composition. A preferred composition consists of, or consists essentially of, a compound of formula (I) and non-active ingredients such as solvents (e.g. water and/or alcohols), colourings, flavourings and sweeteners.

In a preferred embodiment, the level of alcohol in the composition comprising formula (I) is low, preferably less that 25% w/v, more preferably less than 20% w/v, yet more preferably less than 10% w/v, for example less than 5% w/v, 3% w/v or less than 1% w/v. In a further preferred embodiment, there is no alcohol present in the composition comprising formula (1). As used herein, the term "alcohol" refers to any alcohol that can be used in oral preparation, preferably ethanol.

The compound of formula (I) can be used at a suitable concentration, which will be apparent to the skilled person. In one embodiment, suitable concentrations of the compound of formula (I) are between 0.01 % (w/v) to 10% (w/v), preferably from 0.1 % (w/v) to 5% (w/v), more preferably from 1% (w/v) to 3% (w/v), for example 2% (w/v).

The most preferred concentration of a compound of formula (I) is 0.2% w/v.

In one embodiment of the invention, a composition comprises, per 100kg, 98.267kg deionised water; 0.2kg delmopinol HCl; 0.01 kg sodium saccharide; 0.02kg herb flavour COD 76634-34; 1.5kg 99.5% ethanol; and 0.003kg sodium hydroxide.

Mechanical agitation, preferably brushing or rubbing the area containing the infected sub-gingival pocket, can be performed simultaneously with or shortly, preferably immediately, after contacting the oral cavity with a compound of formula (I).

A kit or pack comprising a compound of formula (I) and instructions for use in the treatment of a sub-gingival infection, together with instructions directing the user to use the compound to treat the infection, is included in the invention.

The invention is further described with reference to the following non-limiting example.

### Example 1

In this example delmopinol was used as a rinse to treat a patient who had been cared for over a 2-year period. She had been diagnosed with Morbus Crohn (Crohn's Disease) and with oral complications presenting as severe gingival lesions and swollen gingiva. She had been treated with antibiotics (Zithromax) in the past to control her oral infections and routinely used Chlorhexidine 0.12% rinse solution to control the recolonization of pathogens. Chlorhexidine is used routinely for the control of gingival infection/inflammations as an adjunct to other oral hygiene measures. There are, however side-effects with Chlorhexidine, i.e. staining of teeth, loss of taste and a burning sensation. The alcohol content in the Chlorhexidine solution may also cause some problems for patients. The potential burning sensation was in this case problematic and the patient was not compliant with Chlorhexidine rinsing.

The treatment routine was changed and she was asked to rinse with 0.2% w/v delmopinol once daily for 14 days (instead of chlorhexidine). She had been given a professional cleaning of her teeth prior to rinsing with Delmopinol but no systemic antibiotics were prescribed. Zithromax was used 6 months prior the current treatment.

The following clinical observations were made:
1. During the two weeks no staining or burning sensation as side-effects were reported by the patient who informed the clinicians that she preferred rinsing with Delmopinol.
2. The extent of gingival inflammation was clinically reduced as evidenced by less bleeding on probing and, by visual inspection, less gingival swelling and the absence of ulcerations.

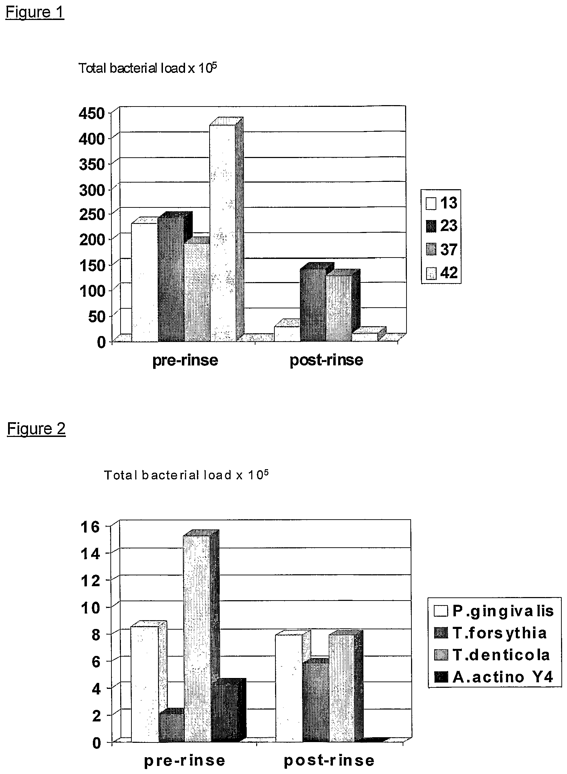

A significant reduction of the total bacterial load from sub-gingival samples taken from four selected sub-gingival pockets (pocket numbers 13, 23, 37 and 42) was seen in samples taken after the 2 week delmopinol period compared to samples taken before the use of delmopinol (see Figure 1).

In addition, a reduction of some key pathogens were found. While the levels of *Porphyromonas gingivalis* and *Tannerella forsythia* were not reduced, the levels of *Treponema denticola* and *Aggregatibacter actinomycetemcomitans* were significantly reduced (*A.actinomycetemcomitans* was in fact eliminated). See Figure 2.

In summary, it appears that rinsing with delmopinol has an advantage over Chlorhexidine for treating subgingival infection. The lack of staining and the lack of burning improve compliance with the rinse. Secondly, rinsing with delmopinol appears to have an overall positive impact on the subgingival microbiota. Delmopinol is therefore clinically useful in controlling a sub-gingival infection.

## Claims

1. Use of a morpholino compound having a general formula (I) 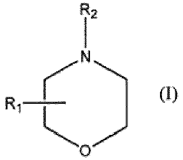 wherein R₁ is a straight or branched alkyl group containing 8 to 16 carbon atoms at the 2- or 3- position of the morpholino ring, and R₂ is a straight or branched alkyl group containing 2 to 10 carbon atoms, substituted with a hydroxy group except in the alpha-position, or a pharmaceutically acceptable salt thereof, in the manufacture of a medicament for the treatment of an infected sub-gingival pocket, wherein the sub-gingival pocket is 4 mm deep or more.

2. Use according to claim 1, wherein the sum of the carbon atoms in the groups R₁ and R₂ of the morpholino compound is at least 10, preferably between 10 and 20.

3. Use according to claim 1 or 2, wherein R₂ of the morpholino compound terminates with the hydroxy group.

4. Use according to any preceding claim, wherein the morpholino compound is 3-(4-propyl-heptyl)-4-(2-hydroxyethyl) morpholine.

5. Use according to any preceding claim, wherein the infected sub-gingival pocket comprises any of *Tannerella forsythensis, Porphyromonas gingivalis, Treponema denticola* or *Actinobacillus actinomycetemcomitans.*

6. Use according to any preceding claim, wherein the sub-gingival infection is **characterised by** periodontitis.

7. Use according to any preceding claim, wherein the sub-gingival infection is **characterised by** chronic periodontitis.

8. Use according to any preceding claim, wherein the medicament further comprises an anti-microbial agent.

9. Use according to any preceding claim, wherein the medicament further comprises an anti-inflammatory agent.

10. Use according to any preceding claim, wherein the medicament is aqueous.

11. Use according to any preceding claim, wherein the medicament is in the form of mouthwash, toothpaste, gel or gum.

12. A kit for use in treating an infection in a sub-gingival pocket that is 4 mm deep or more, comprising a compound as defined in any of the claims 1 to 4 and instructions that the compound is to be used for treatment of the infection

## Patentansprüche

1. Verwendung einer Morpholinoverbindung mit der allgemeinen Formel (I) 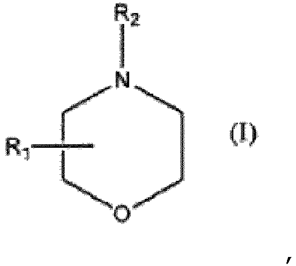 wobei R₁ für eine geradkettige oder verzweigte Alkylgruppe mit 8 bis 16 Kohlenstoffatomen in der 2-oder 3-Stellung des Morpholinrings steht und R₂ für eine geradkettige oder verzweigte Alkylgruppe mit 2 bis 10 Kohlenstoffatomen, substituiert durch eine Hydroxygruppe, ausgenommen in der alpha-Stellung, steht, oder ein pharmazeutisch unbedenkliches Salz davon zur Herstellung eines Medikaments zur Behandlung einer infizierten subgingivalen Tasche, wobei die subgingivale Tasche 4 mm tief oder tiefer ist.

2. Verwendung nach Anspruch 1, wobei die Summe der Kohlenstoffatome in den Gruppen R₁ und R₂ der Morpholinoverbindung mindestens 10, vorzugsweise zwischen 10 und 20, beträgt.

3. Verwendung nach Anspruch 1 oder 2, wobei R₂ der Morpholinoverbindung in einer Hydroxygruppe endet.

4. Verwendung nach einem der vorhergehenden Ansprüche, wobei es sich bei der Morpholinoverbindung um 3-(4-Propylheptyl)-4-(2-hydroxyethyl)morpholin handelt.

5. Verwendung nach einem der vorhergehenden Ansprüche, wobei die infizierte subgingivale Tasche *Tannerella forsythensis, Porphyromonas gingivalis, Treponema denticola* oder *Actinobacillus actinomycetemcomitans* umfasst.

6. Verwendung nach einem der vorhergehenden Ansprüche, wobei die subgingivale Infektion durch Parodontitis charakterisiert ist.

7. Verwendung nach einem der vorhergehenden Ansprüche, wobei die subgingivale Infektion durch chronische Parodontitis charakterisiert ist.

8. Verwendung nach einem der vorhergehenden Ansprüche, wobei das Medikament weiterhin ein antimikrobielles Mittel umfasst.

9. Verwendung nach einem der vorhergehenden Ansprüche, wobei das Medikament weiterhin ein entzündungshemmendes Mittel umfasst.

10. Verwendung nach einem der vorhergehenden Ansprüche, wobei das Medikament wässrig ist.

11. Verwendung nach einem der vorhergehenden Ansprüche, wobei das Medikament in Form eines Mundwassers, einer Zahnpasta, eines Gels oder eines Gummis vorliegt.

12. Kit zur Verwendung bei der Behandlung einer Infektion in einer subgingivalen Tasche, die 4 mm tief oder tiefer ist, umfassend eine wie in einem der Ansprüche 1 bis 4 definierte Verbindung und Anweisungen, dass die Verbindung zur Behandlung der Infektion zu verwenden ist.

## Revendications

1. Utilisation d'un composé morpholino répondant à une formule générale (I) 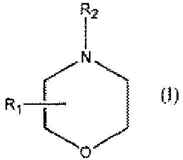 où R₁ représente un groupement alkyle linéaire ou ramifié comportant entre 8 et 16 atomes de carbone en position 2 ou 3 du cycle morpholino, et R₂ représente un groupement alkyle linéaire ou ramifié comportant entre 2 et 10 atomes de carbone, substitué par un groupement hydroxy excepté en position alpha, ou sel pharmaceutiquement acceptable de celui-ci, dans la fabrication d'un médicament destiné au traitement d'une poche subgingivale infectée, où la poche subgingivale est profonde de 4 mm ou plus.

2. Utilisation selon la revendication 1, où le nombre total d'atomes de carbone des groupements R₁ et R₂ du composé morpholino est d'au moins 10, préférentiellement entre 10 et 20.

3. Utilisation selon la revendication 1 ou 2, où le radical R₂ du composé morpholino est terminé par le groupement hydroxy.

4. Utilisation selon l'une quelconque des revendications précédentes, où le composé morpholino est la 3-(4-propyl-heptyl)-4-(2-hydroxyéthyl)morpholine.

5. Utilisation selon l'une quelconque des revendications précédentes, où la poche subgingivale infectée comprend l'un quelconque des membres du groupe constitué par *Tannerella forsythensis, Porphyromonas gingivalis, Treponema denticola* ou *Actinobacillus actinomycetemcomitans.*

6. Utilisation selon l'une quelconque des revendications précédentes, où l'infection subgingivale est **caractérisée par** une parodontite.

7. Utilisation selon l'une quelconque des revendications précédentes, où l'infection subgingivale est **caractérisée par** une parodontite chronique.

8. Utilisation selon l'une quelconque des revendications précédentes, où le médicament comprend en outre un agent antimicrobien.

9. Utilisation selon l'une quelconque des revendications précédentes, où le médicament comprend en outre un agent anti-inflammatoire.

10. Utilisation selon l'une quelconque des revendications précédentes, où le médicament est aqueux.

11. Utilisation selon l'une quelconque des revendications précédentes, où le médicament se présente sous la forme d'un bain de bouche, d'une pâte dentaire, d'un gel ou d'une gomme.

12. Kit pour utilisation dans le traitement d'une infection dans une poche subgingivale profonde de 4 mm ou plus, comprenant un composé selon l'une quelconque des revendications 1 à 4 et des instructions d'utilisation du composé dans le traitement de l'infection.
